# EUROPEAN PATENT SPECIFICATION

(11) **EP 0 644 178 B1**
(45) Date of publication and mention of the grant of the patent: **02.04.1997**
(21) Application number: 93118735.5
(22) Date of filing: 22.11.1993
(51) Int. Cl.: C07C 209/84

(54) **Process for ethalfluralin purifying**
Verfahren zur Reinigung von Ethalfluralin
Procédé de purification d'éthalfluralin

(30) Priority: 13.09.1993 IT TO930667
(43) Date of publication of application: 22.03.1995
(73) Proprietor: FINCHIMICA S.p.A., 25025 Manerbio (Brescia) (IT)
(72) Inventor: Donadello, Graziello, I-36078 Valdagno (Vicenza) (IT)
(74) Representative: Rambelli, Paolo

(56) References cited:
- EP-A- 0 151 725
- EP-A- 0 479 384
- US-A- 4 226 789
- US-A- 4 874 895

## Description

The present invention relates to a process for purifying crude ethalfluralin, which contains trace amounts of nitrosamines, in order to reduce their amount to a non-dangerous level and particularly to a value lower than 0.5 ppm.

Ethalfluralin is the commercial name of a herbicide, which is widely used in agriculture and whose chemical name is 4-trifluoromethyl-2,6-dinitro-N-ethyl-N-methallylaniline or (IUPAC) N-ethyl- alpha-alpha-alpha-trifluoro-N-(methylallyl)-2,6-dinitro-p-toluidine.

Ethalfluralin belongs to a wide class of dinitro anilines having herbicidal activity and in such a class is characterized by the presence of an unsaturated methallyl or 2-methyl-2-propenyl group bound to the amino group.

It is known that crude dinitroanilines contain small amounts of nitrosamines, typically from a few ppm to a few hundred ppm. It is assumed that the origin of nitrosamines in dinitroanilines derives from the final stage of their conventional preparation process, which consists of a reaction between the dinitro-chloro-benzene derivative with the desired secondary amine; the dinitro-chloro-benzene derivative may contain small amounts of nitrosating agents, such as nitrogen oxides which are present as the result of the nitration reaction, which may react with the amine to form the nitrosamines, whose presence in the dinitroanilines is considered undesirable due to the discovery of their carcinogenic properties in animals.

Following the development of highly sensitive methods of analysis it is possible to ascertain the presence of nitrosamines in the dinitroanilines down to a level of 0.02 ppm and the maximum value which is desired for their level in a commercial product is presently defined at 0.5 ppm.

In order to reduce the amount of nitrosamines, several purification processes have been proposed. Particularly, U.S. patent No.4,226,789 describes a process of treatment of dinitroanilines with HCl in an aqueous solution, with a concentration of at least 20% or with gaseous HCl under low pressure. With reference specifically to ethalfluralin, the cited U.S. patent teaches the need of using gaseous hydrochloric acid under pressure, since the use of an aqueous solution of hydrochloric acid causes addition reactions across the methallyl double-bond with the consequent formation of the chloro derivative and reduction of the title of ethalfluralin and of the overall process yield.

Furthermore, the compound of addition of hydrochloric acid on ethalfluralin is stable and its conversion to ethalfluralin by means of a treatment with sodium hydroxide is not possible.

The treatment with gaseous hydrochloric acid may not be conveniently carried out as an industrial process, since it involves substantial difficulties relating to the availability on the market and preparation of the gaseous hydrochloric acid and a substantial increase in the plant investment costs.

U.S. patent No.4,874,895 describes a process for purification of trifluralin (4-trifluoromethyl-2,6-dinitro-N,N-di-n-propylaniline) with the use of aqueous hydrobromic acid in the presence of additives consisting of sulfamic acid and bisulfite. The disclosed process is advantaqeous, since adapted not only to the removal of the nitrosamines, but also to avoid the growth of nitrosamines in the purified trifluralin. Moreover, the process allows the almost quantitative recovery of the treatment solution and its recycling for several purification cycles.

As a difference from the several trifluralin purification processes which are described in literature, wherein the applicability of the process is generally broadened to the whole class of the dinitroanilines, the process disclosed by the quoted U.S. patent is strictly limited to trifluralin; in view of the teaching provided by U.S. patent No.4,226,789, the treatment with aqueous halogenhydric acids was not considered as industrially applicable for the purification of unsaturated dinitroanilines.

Surprisingly, it has been found that aqueous hydrobromic acid, under the process condition adopted for the treatment for removal of nitrosamines from ethalfluralin, has a non-analogous behaviour to that of hydrochloric acid, since it is not susceptible to generate the formation of products of addition across the double-bond present in ethalfluralin. In view of such a discovery, the subject of the present invention is a process for the purification of the unsaturated dinitroaniline, crude ethalfluralin, by treatment with aqueous halogenhydric acid, suitable to reduce the therein contained nitrosamine level. to a value lower than 0.5 ppm, while avoiding the formation of compounds of addition of the halogenhydric acid on the unsaturated bond, characterized in that crude ethalfluralin is treated with an aqueous solution of hydrobromic acid in the presence of sulfamic acid and of a sulfur compound selected from the group consisting of bisulfites, metabisulfites, hydrosulfites, sulfurous acid and gaseous sulfur dioxide.

The process of the invention allows to reduce the amount of nitrosamines down to the desired level, without reduction of the title of ethalfluralin.

Typically, the treatment with the aqueous hydrobromic acid solution is carried out at a temperature from 20 to 90°C, preferably from 60°C and 80°C for a time from 10 min. to 4 h.

The hydrobromic acid concentration in the aqueous solution is typically between 15 and 50% by wt., preferably between 23 and 27% by wt..

The latter concentration range is preferred as to the more favourable kinetics, the greater ease of separation between the aqueous phase and the ethalfluralin phase at the end of the treatment and as to the possibility of using again for several recyclings the solution which, when exhausted, may eventually be regenerated by distillation. The distillation of an aqueous solution of hydrobromic acid having a concentration in the range from 23 to 27% by wt. leads to obtain an azeotropic mixture having a concentration of hydrobromic acid of 48% wt. which may be easily re-used in the process by being diluted with the optional addition of fresh hydrobromic acid.

The ratio of the aqueous solution of hydrobromic acid and ethalfluralin is typically between 0.2 and 0.8 l of aqueous acidic solution per kg of ethalfluralin and preferably from 0.3 to 0.5 l/kg.

The amount of sulfamic acid is typically from 0.1 to 3 g/l referred to the hydrobromic acid solution and preferably from 0.4 to 0.7 g/l. Sulfamic acid is preferably added as an aqueous solution having a concentration from 10 to 20% by wt. which allows a simple dosage to achieve the above-mentioned concentration of sulfamic acid in the hydrobromic acid solution.

The sulfur compound is selected from bisulfites, metabisulfites, hydrosulfites, preferably of alkali metals, particularly of sodium, sulfurous acid and gaseous sulfur dioxide and is added in the amount corresponding to a content of sulfur dioxide of from 0.3 to 1.2 g/l referred to the aqueous hydrobromic acid solution.

Sodium bisulfite is preferred, since it may be used in the commercially available forms such as anhydrous sodium bisulfite having a content of SO₂ of about 60% by wt. or as a solution of sodium bisulfite with a content of SO₂ from 24 to 26% by wt. or as sodium metabisulfite having a content of SO₂ of about 65% by wt..

According to the process of the invention, crude ethalfluralin is mixed under strong agitation with the aqueous hydrobromic acid solution to which sulfamic acid and the sulfur compound have been added. The molten organic phase is then separated from the aqueous hydrobromic acid solution and the refined ethalfluralin is recovered from the molten organic phase by washing - while still hot - with alkaline water solution to neutralize the pH; thereafter, the molten mass is solidified by cooling.

The aqueous phase of hydrobromic acid separated from the molten organic phase is then added again with sulfamic acid and sulfur compound at a temperature higher than about 50°C and may then be re-used for the treatment of a new batch of ethalfluralin. The aqueous solution may be recycled up to twenty times, without reduction of its effectiveness in reducing the level of nitrosamines to a value below 0.5 ppm and without the occurrence of reaction of addition on the double-bond. The exhausted hydrobromic acid solution may then be re-generated by distillation.

The thus obtained ethalfluralin is furthermore extremely stable against the formation of nitrosamines during the thermal treatment to which it is subjected for the preparation of herbicidal compositions.

### Example 1

### Preparation of ethalfluralin

960 g water and 810 g 4-chloro-3,5-dinitro-benzotrifluoride are introduced into a glass balloon having a volume of 3000 ml placed in a thermostatic bath and provided with a stirrer, reflux condensor, thermometer and charging funnel.

297 g N-ethylmethallylamine are added dropwise under strong stirring and while maintaining the temperature below 40°C.

At the end of the addition, the temperature of the reaction mixture is maintained at 35-40°C for 20 min. and then 405 g of a 30% sodium hydroxide aqueous solution are added, while maintaining the temperature at 40-45°C. At the end of the addition of sodium hydroxide, the reaction mixture is heated to 60°C and then to 70°C in about 1 h. and this temperature is maintained for 30 min..

Then, 405 g water are added, while maintaining the temperature at 70°C; at the end of the water addition, the reaction mixture is maintained at 70°C for 30 min.; the pH is adjusted to 6.5 by addition of 7 g of 36% HCl.

The stirrer is stopped and the reaction mixture is allowed to clear at 60-70°C and then the two phases are separated. The lower phase consists of ethalfluralin and weighs 1000 g.

The gas chromatographic analysis shows a title higher than 95% and the absence of the product of addition across the double-bond.

The GC/TEA analysis shows a nitrosamine content of 47 ppm.

### Example 2 (comparison)

### Removal of nitrosamines contained in Ethalfluralin with 36% hydrochloric acid

25 g of an aqueous solution of 36% HCl are added to 200 g of ethalfluralin containing 47 ppm of nitrosamines in a balloon provided with a stirrer and heated to 70°C under stirring for 1 h..

The stirrer is stopped and the two layers are allowed to separate. The organic layer is poured dropwise into a balloon containing 50 ml water and 5 ml aqueous solution of 10% Na₂CO₃. The temperature is maintained at 60-70°C under stirring and the pH, which must be higher than 7, is controlled. Stirring is maintained for 15 min. and the reaction mixture is allowed to clear.

The organic phase is separated and analyzed. The nitrosamines content by GC/TEA analysis is lower than 0.2 ppm. The gas chromatrographic analysis of the product shows a content of the product of addition across the double-bond of 2.0%.

In a second test, carried out according to the same procedure but with the use of 50 g of 36% HCl and for a time of 2 h., a product is obtained having a nitrosamines content such that it is not determined by GC/TEA analysis (the sensitivity threshold of the instrument is of 0.05 ppm), however with a content of the product of the addition across the double-bond higher than 5%.

### Example 3

### Nitrosamines removal with 25% hydrobromic acid

600 g of crude ethalfluralin containing 47 ppm of nitrosamines are mixed in a glass balloon, provided with stirrer and reflux condensor, with 200 g of an aqueous solution of 25% HBr, 0.1 g sulfamic acid and 0.1 ml of a 30% sodium bisulfite aqueous solution.

The reaction mixture is heated to 80°C under stirring and maintained at that temperature for 3 h.. The stirrer is stopped and the reaction mixture is allowed to clear. The organic layer is separated and is poured dropwise into a balloon containing 200 ml water and 10 ml of a 10% Na₂CO₃ aqueous solution and stirred at 60-70°C for 15 min.. The stirrer is stopped and the two phases are allowed to separate.

The organic layer is analyzed and shows by GC/TEA analysis a nitrosamines content lower than 0.2 ppm (sensitivity threshold: 0.05 ppm). The gas chromatographic analysis shows a title higher than 95% and the absence of the product of addition across the double-bond.

## Claims

1. A process for the preparation of the unsaturated dinitroaniline, ethalfluralin, having a level of nitrosamines lower than 0.5 ppm and essentially free from the compound of addition of halogenhydric acid on the methallyl double-bond, by treatment with aqueous halogenhydric acid, characterized in that the ethalfluralin is treated under agitation with an aqueous solution of hydrobromic acid in the presence of sulfamic acid and of a sulfur compound selected from the groups consisting of bisulfites, metabisulfites, hydrosulfites, sulfurous acid and gaseous sulfur dioxide.

2. A process according to claim 1, wherein the aqueous hydrobromic acid solution has a concentration from 15 to 50% by wt..

3. A process according to claim 2, wherein the aqueous hydrobromic acid solution has a concentration from 23 to 27% by wt..

4. A process according to any of claims 1 to 3, wherein the ratio between the aqueous hydrobromic acid solution and crude ethalfluralin is from 0.2 to 0.8 l/kg, preferably from 0.4 to 0.6 l/kg.

5. A process according to any of claims 1 to 4, wherein the amount of sulfamic acid added to the hydrobromic acid solution is from 0.1 to 3 g/l, preferably from 0.4 to 0.7 g/l.

6. A process according to any of claims 1 to 5, wherein the amount of the sulfur compound added to the hydrobromic acid solution corresponds to a content of sulfur dioxide from 0.3 to 1.2 g/l, preferably from 0.5 to 0.8 g/l.

## Patentansprüche

1. Verfahren zur Herstellung des ungesättigten Dinitroanilins Ethalfluralin, das eine Konzentration an Nitrosaminen von weniger als 0,5 ppm aufweist und im wesentlichen frei von der Verbindung der Anlagerung von Halogenwasserstoffsäure an die Methallyl-Doppelbindung ist, durch Behandlung mit wässeriger Halogenwasserstoffsäure, dadurch gekennzeichnet, daß rohes Ethalfluralin unter Rühren mit einer wässerigen Lösung von Bromwasserstoffsäure in Gegenwart von Sulfamidsäure und einer Schwefelverbindung, ausgewählt aus den Gruppen bestehend aus Hydrogensulfiten, Disulfiten, Dithioniten, schwefliger Säure und gasförmigem Schwefeldioxid, behandelt wird.

2. Verfahren nach Anspruch 1, wobei die wässerige Bromwasserstoffsäurelösung eine Konzentration von 15 bis 50 Gew.% aufweist.

3. Verfahren nach Anspruch 2, wobei die wässerige Bromwasserstoffsäurelösung eine Konzentration von 23 bis 27 Gew.% aufweist.

4. Verfahren nach einem der Ansprüche 1 bis 3, wobei das Verhältnis zwischen der wässerigen Bromwasserstoffsäurelösung und rohem Ethalfluralin 0,2 bis 0,8 l/kg, vorzugsweise 0,4 bis 0,6 l/kg, beträgt.

5. Verfahren nach einem der Ansprüche 1 bis 4, wobei die Menge an Sulfamidsäure, die der Bromwasserstoffsäurelösung zugesetzt wird, 0,1 bis 3 g/l, vorzugsweise 0,4 bis 0,7 g/l, beträgt.

6. Verfahren nach einem der Ansprüche 1 bis 5, wobei die Menge der Schwefelverbindung, die der Bromwasserstoffsäurelösung zugesetzt wird, einem Schwefeldioxid-Gehalt von 0,3 bis 1,2 g/l, vorzugsweise 0,5 bis 0,8 g/l, entspricht.

## Revendications

1. Procédé de préparation de la dinitroaniline insaturée, éthalfluraline, ayant une teneur en nitrosamine inférieure à 0,5 ppm et essentiellement exempte de composé d'addition d'acide halogénohydrique sur la double liaison méthallyle, par traitement avec une solution aqueuse d'acide halogénohydrique caractérisé en ce qu'on traite l'éthalfluraline brute sous agitation avec une solution aqueuse d'acide bromhydrique en présence d'acide sulfamique et d'un composé de soufre choisi dans les groupes constitués des bisulfites, métabisulfites, hydrosulfites, acides sulfureux et dioxyde de soufre gazeux.

2. Procédé selon la revendication 1, où la solution aqueuse d'acide bromhydrique a une concentration de 15 à 50% en poids.

3. Procédé selon la revendication 2, où la solution d'acide bromhydrique aqueuse a une concentration de 23 à 25% en poids.

4. Procédé selon l'une quelconque des revendications 1 à 3, où le rapport entre la solution aqueuse d'acide bromhydrique et l'éthalfluraline brute est de 0,2 à 0,8 l/kg, de préférence de 0,4 à 0,6 l/kg.

5. Procédé selon l'une quelconque des revendications 1 à 4, où la quantité d'acide sulfamique ajouté à la solution d'acide bromhydrique est de 0,1 à 3 g/l, de préférence de 0,4 à 0,7 g/l.

6. Procédé selon l'une quelconque des revendications 1 à 5, où la quantité de composé de soufre ajouté à la solution d'acide bromhydrique correspond à une teneur en dioxyde de soufre de 0,3 à 1,2 g/l de préférence de 0,5 à 0,8 g/l.
